Europäisches Patentamt

European Patent Office

Office européen des brevets

(19)

(11) Veröffentlichungsnummer: **0 511 526 A1**

## (12) EUROPÄISCHE PATENTANMELDUNG

(21) Anmeldenummer: **92106039.8**

(22) Anmeldetag: **08.04.92**

(51) Int. Cl.⁵: **C12P 41/00**, C12P 7/40

(30) Priorität: **29.04.91 AT 886/91**

(43) Veröffentlichungstag der Anmeldung:
**04.11.92 Patentblatt 92/45**

(84) Benannte Vertragsstaaten:
**AT BE CH DE DK ES FR GB IT LI NL SE**

(71) Anmelder: **Chemie Linz Gesellschaft m.b.H.**
**St.Peter-Strasse 25**
**A-4021 Linz(AT)**

(72) Erfinder: **Buchner, Maria, Dipl.-Ing.**
**Ferdinand Markl-Strasse 39**
**A-4040 Linz(AT)**
Erfinder: **Estermann, Robert**
**Reischekstrasse 25**
**A-4020 Linz(AT)**
Erfinder: **Mayrhofer, Herbert**
**Freistädter-Strasse 42**
**A-4210 Engerwitzdorf(AT)**
Erfinder: **Banko, Gerald, Dr.**
**Kefergutstrasse 26**
**A-4020 Linz(AT)**

(74) Vertreter: **Kunz, Ekkehard, Dr.**
**Chemie Linz GesmbH., Patentwesen, St.**
**Peter Strasse 25**
**A-4021 Linz(AT)**

(54) **Verfahren zur enzymatischen Hydrolyse eines Carbonsäurederivates.**

(57)  Verfahren zur enzymatischen Hydrolyse eines Carbonsäurederivates durch Lösen des Carbonsäurederivates in einem organischen Lösungsmittel, das mit Wasser nur in einem geringen Ausmaß mischbar ist, Sättigung der organischen Lösung mit Wasser, Inkontaktbringen der wassergesättigten organischen Lösung mit einer Hydrolase, wobei die Hydrolyse stattfindet, worauf die Reaktionslösung wieder mit Wasser gesättigt und solange wieder mit der Hydrolase und anschließend mit Wasser in Kontakt gebracht wird, bis der erwünschte Umsetzungsgrad erreicht ist.

EP 0 511 526 A1

Die Erfindung betrifft ein Verfahren zur enzymatischen Hydrolyse eines Carbonsäurederivates in einem organischen Lösungsmittel.

Eine große Anzahl von Enzymen ist befähigt, Carbonsäurederivate mehr oder weniger spezifisch zu hydrolisieren. Diese Eigenschaft der Hydrolasen wird seit langem wirtschaftlich genutzt. Dabei ist zu beachten, daß Hydrolasen normalerweise in wäßrigen Lösungen gute Umsetzungsraten und im allgemeinen geringe Aktivitätsverluste zeigen, daß sie jedoch in Wasser löslich und nur selten aus wäßrigen Reaktionslösungen zurückzugewinnen und erneut einsetzbar sind. Kovalent immobilisierte Hydrolasen sind zwar aus wäßrigen Lösungen wiedergewinnbar, sie weisen jedoch geringere Umsetzungsraten und schlechtere Aktivitäten auf, als eine gleiche Mengen Hydrolase, die in nicht immobilisierter Form eingesetzt wird. Zusätzlich wird aus Enzymimmobilisaten in wäßrigen Systemen immer auch Enzym herausgelöst.

Es wurde daher auch schon versucht, enzymatische Hydrolysen in organischem Medium durchzuführen. So ist in Pakistan Journal of Biochemistry, Vol.10, Nr. 2, 1976 geoffenbart, daß bei der Hydrolyse von Carbonsäureestern die anfängliche Hydrolysegeschwindigkeit in organischem Medium manchmal höher sein kann, als in wäßrigem Medium, allerdings sinkt die Aktivität der Hydrolase im organischen Medium rasch ab, da Hydrolasen gegenüber organischen Lösungsmitteln empfindlich sind.

In Chemical Abstracts Vol.112, 154387z wird daher vorgeschlagen, Hydrolasen chemisch zu modifizieren, um deren Toleranz gegenüber organischen Lösungsmitteln zu erhöhen.

In Chemical Abstracts Vol.109, 188832n ist eine enzymatische Hydrolyse mit einer chemisch modifizierten Hydrolase in wassergesättigtem Benzol beschrieben.

Es wurde nun unerwarteterweise gefunden, daß es für gute Umsetzungsraten und gleichbleibend hohe Aktivität einer Hydrolase in einem organischen Lösungsmittel nicht notwendig ist, die Hydrolase chemisch zu modifizieren, wenn man ein organisches Lösungsmittel verwendet, das mit Wasser nur in geringem Ausmaß mischbar ist und wenn man dafür sorgt, daß das organische Lösungsmittel im Verlauf der Hydrolyse, bei der Wasser verbraucht wird, mit Wasser gesättigt bleibt.

Gegenstand der Erfindung ist daher ein Verfahren zur enzymatischen Hydrolyse eines Carbonsäurederivates, das dadurch gekennzeichnet ist, daß das Carbonsäurederivat in einem organischen Lösungsmittel, das mit Wasser nur in einem geringen Ausmaß mischbar ist, gelöst wird, worauf die Lösung mit Wasser gesättigt und mit einer Hydrolase in Kontakt gebracht wird, wobei eine Hydrolyse unter Wasserverbrauch stattfindet, worauf die organische Reaktionslösung solange erneut mit Wasser gesättigt und mit der Hydrolase in Kontakt gebracht wird, bis der gewünschte Umsetzungsgrad erreicht ist.

Das erfindungsgemäße Verfahren ist zur Hydrolyse von Carbonsäurederivaten, die mit Hilfe einer Hydrolase enzymatisch hydrolisierbar sind, geeignet. Solche Carbonsäurederivate sind beispielsweise Carbonsäureester, -diester, -triester, Carbonsäureamide, Carbonsäurethioester usw. oder deren analoge Thiocarbonsäurederivate.

Eine besondere Bedeutung hat das Verfahren für die Hydrolyse von Carbonsäurederivaten, die im Säureteil oder im Derivatteil Substituenten tragen, die ein chirales Zentrum im Molekül bedingen oder von Carbonsäurederivaten, bei denen durch die Hydrolyse ein chirales Zentrum entsteht. Solche chirale oder prochirale Carbonsäurederivate können mit Hilfe einer stereospezifischen Hydrolase in optisch aktive Verbindungen hydrolisiert werden, in denen eines der möglichen Enantiomeren je nach der Stereospezifität der Hydrolase zumindet angereichert vorliegt, wobei unter chiralen Carbonsäurederivaten sowohl racemische Gemische als auch Gemische, in denen eines der möglichen Enantiomeren zumindest angereichert vorliegt, zu verstehen sind.

Bevorzugt sind unter Carbonsäurederivate Mischungen von Enantiomeren chiraler Carbonsäureester, die das chirale Zentrum im Säureteil aufweisen, besonders bevorzugt 2-substituierte Alkansäureester, ganz besonders bevorzugt 2-Halogenpropionsäureester, zu verstehen.

Als Hydrolyseprodukte entstehen dementsprechend Carbonsäuren oder Thiocarbonsäuren und Alkohole, Amine, Thiole, usw. wobei entweder die Carbonsäuren oder die Alkohole, Amine, Thiole usw. oder auch beide als erwünschte Reaktionsprodukte vorgesehen sein und gewonnen werden können.

Zur Durchführung des erfindungsgemäßen Verfahrens wird zuerst ein Carbonsäurederivat in einem organischen Lösungsmittel, das mit Wasser nur in einem geringen Ausmaß mischbar ist, gelöst.

Als organisches Lösungsmittel dienen z.B. Kohlenwasserstoffe, wie Pentan, Hexan, Benzol, Toluol, halogenierte Kohlenwasserstoffe, wie Methylenchlorid, Chloroform, Tetrachlorkohlenstoff, Ethylenchlorid, Chlorbenzole, oder Ether, wie Diethylether, Diisopropylether, oder Mischungen solcher Lösungsmittel. Bevorzugte Lösungsmittel sind Ether, insbesondere Diisopropylether. Die Auswahl des Lösungsmittels kann dabei von Bedeutung sein, da die Reaktion in einem bestimmten Lösungsmittel schneller ablaufen kann, als in einem anderen. Es kann unter Umständen von Vorteil sein, dem organischen Lösungsmittel eine kleine Menge eines organischen Co-Lösungsmittels, das mit Wasser mischbar ist, wie etwa einen Alkohol, z. B. Methanol, Ethanol, Isopropanol, ein Keton, z. B. Aceton usw. zuzugeben, um die Löslichkeit des Carbonsäurederivates im organischen Lösungsmittel zu erhöhen. Die zugegebene Menge des Co-Lösungsmittels muß aber so gering sein, daß das organische Lösungsmittel durch Zugabe des Co-Lösungsmittels nicht völlig mit Wasser mischbar wird. Das Lösungsmittel für eine gewünschte Umsetzung ist für den Fachmann durch einfache Vorversuche leicht zu finden.

Die Lösung der Ausgangsverbindung in dem organischen Lösungsmittel wird so konzentriert als möglich hergestellt, wobei die Konzentration des Ausgangsproduktes im Lösungsmittel vom jeweiligen Ausgangsprodukt und vom jeweils verwendeten Lösungsmittel abhängig ist.

Die organische Lösung wird sodann mit Wasser gesättigt.

Zur Sättigung mit Wasser wird die Lösung entweder mit einem System, das gebundenes Wasser enthält und das befähigt ist, dieses Wasser beim Kontakt mit einem organischen Lösungsmittel abzugeben, oder mit einer wäßrigen Phase in Kontakt gebracht.

Systeme, die gebundenes Wasser enthalten, sind beispielsweise wasserenthaltende Hydrogele, beispielsweise Polyacrylamidgele, Polysaccharidgele usw.. Der organischen Lösung wird eine solche Menge an gebundenem Wasser zugegeben, die ausreicht, um die Lösung mit Wasser zu sättigen, wobei unter Wasser sowohl reines Wasser als auch Puffer- oder Salzlösungen zu verstehen sind. Die Menge der verwendeten Hydrogele hängt dabei von der Wasseraufnahmefähigkeit sowohl des Hydrogels als auch des organischen Lösungsmittels ab.

Als wäßrige Phase kommt reines Wasser, eine Pufferlösung oder auch eine wäßrige Salzlösung in Betracht. Als Pufferlösung wird zweckmäßig eine solche eingesetzt, in der die Hydrolase hohe Umsetzungsraten und hohe Spezifität zeigt. Zur Sättigung mit Wasser wird die organische Lösung direkt in die wäßrige Phase eingebracht oder mit einer wäßrigen Phase vermischt und absetzen gelassen, wobei sich 2 Phasen bilden.

Die wassergesättigte Lösung wird anschließend mit einer Hydrolase in Kontakt gebracht.

Als Hydrolasen kommen je nach Ausgangsverbindung und erwünschtem Produkt für die jeweilige Umsetzung geeignete Hydrolasen in Betracht. Beispiele für Hydrolasen sind etwa Esterasen, Proteasen, Amidasen, usw. Zweckmäßig wird je nach gewünschter Umsetzung eine solche Hydrolase eingesetzt, die die Umsetzung möglichst spezifisch, im Falle chiraler oder prochiraler Carbonsäurederivate möglichst stereospezifisch ausführt. Bevorzugt wird im erfindungsgemäßen Verfahren eine Lipase verwendet. Ein Vorteil des erfindungsgemäßen Verfahrens ist es, daß die Hydrolase nicht chemisch modifiziert werden muß, um deren Toleranz gegen chemische Lösungsmittel zu erhöhen. Es können im erfindungsgemäßen Verfahren aber auch chemisch modifizierte Hydrolasen eingesetzt werden.

Die Hydrolase kann als solche, adsorbiert an einen Träger, etwa an Celite, Kieselgel, Staub, Glasperlen oder auch immobilisiert eingesetzt werden. Bevorzugt wird die Hydrolase adsorbiert an einen inerten Träger, besonders bevorzugt an Celite eingesetzt, wobei es zur Adsorption der Hydrolase an Celite genügt, die Hydrolase einfach mit Celite zu vermischen.

Ein wesentlicher Vorteil des Verfahrens ist, daß die Hydrolase nicht immobilisiert vorgelegt werden muß, da sie in organischen Lösungsmitteln nicht löslich ist.

Wurde zur Wassersättigung der organischen Lösung ein wasserenthaltendes Hydrogel verwendet, wird die Hydrolase direkt in die wassergesättigte Lösung, in der sich auch das Hydrogel befindet, zugegeben. Beim Kontakt der wassergesättigten Lösung mit der Hydrolase findet die Hydrolyse im enzymspezifischen Ausmaß und mit enzymspezifischer Selektivität statt, wobei das bei der Hydrolyse verbrauchte Wasser aus dem Hydrogel nachgeliefert wird, sodaß die organische Reaktionslösung stets wassergesättigt bleibt.

Wurde ein chirales oder prochirales Carbonsäurederivat eingesetzt, wird die Reaktion bis zu einem gewünschten Umsetzungsgrad, der durch Bestimmung des optischen Drehwertes ermittelt werden kann, ablaufen gelassen.

Nach Erreichen des gewünschten Umsetzungsgrades werden das Enzym und das Hydrogel abfiltriert. Die Lösung kann dann je nach gewünschtem Produkt wie üblich aufgearbeitet werden, wobei das gewünschte Produkt mit Hilfe von Extraktion, Umkristallisieren, Destillation oder Chromatographie wie üblich erhalten und/oder gereinigt werden kann.

Wurde zur Sättigung der organischen Lösung mit Wasser eine wäßrige Phase verwendet, wird die Hydrolase in einen Reaktionsbehälter, beispielsweise in eine Säule eingebracht und die wassergesättigte, organische Lösung durch den Behälter und über die Hydrolase gepumpt, sodaß die Hydrolase nicht mit der wäßrigen Phase in Berührung kommt.

Beim Kontakt der organischen wassergesättigten Lösung mit der Hydrolase findet die Hydrolyse in enzymspezifischem Ausmaß und mit enzymspezifischer Stereoselektivität statt. Dabei wird die wassergesättigte Lösung kontinuierlich über die Hydrolase und anschließend durch die wäßrige Phase geleitet, da beim einmaligen Kontakt der Hydrolase mit der Reaktionslösung der gewünschte Umsetzungsgrad im allgemeinen nicht erreicht wird. Da Hydrolasen im allgemeinen beide Enantiomeren eines optisch aktiven Carbonsäurederivates umsetzen können, wobei sie aber ein Enantiomer bevorzugt umsetzen, ist es im Fall chiraler oder prochiraler Carbonsäurederivate im allgemeinen angebracht, den optischen Drehwert der Reaktionslösung, der ein Maß für den jeweiligen Enantiomerenüberschuß darstellt, kontinuierlich zu messen und die Reaktion nach Umsetzung des von der Hydrolase bevorzugten Enantiomeren abzubrechen, um möglichst enantiomerenreine Produkte zu erhalten.

Beim kontinuierlichen Pumpen der organischen, wassergesättigten Lösung über das Enzym ist darauf zu achten, daß die Lösung beim Kontakt mit dem Enzym stets wassergesättigt ist. Da bei der Hydrolyse pro Mol gespaltener Bindung ein Mol Wasser verbraucht wird, muß dieses Wasser ersetzt werden, bevor die organische Lösung erneut mit der Hydrolase in Kontakt kommt.

Das verbrauchte Wasser kann etwa dadurch ersetzt werden, daß die organische Lösung nach dem Kontakt mit der Hydrolase über basische, Hydroxidionen enthaltende Agentien, die sich z.B. in einer Säule befinden, geleitet wird. Dabei bildet sich pro Mol Carbonsäure 1 Mol Carbonsäuresalz und 1 Mol Wasser, sodaß das bei der Hydrolyse verbrauchte Wasser ersetzt wird und die Lösung wassergesättigt bleibt. Als basische Agentien können z.B. Ionenaustauscher in der OH-Form, Alkali- oder Erdalkalihydroxide verwendet werden.

Nach Verlassen der Säule enthält die Lösung keine bei der enzymatischen Reaktion gebildete Carbonsäure mehr, da diese an die basischen Agentien gebunden bleibt, ist aber durch die Bildung von einem Mol Wasser pro Carbonsäuresalz wieder mit Wasser gesättigt. Die Reaktionslösung wird kontinuierlich solange über die Hydrolase und anschließend über die Säule mit den Hydroxidionen enthaltenden Agentien gepumpt, bis der gewünschte Umsetzungsgrad erreicht ist. Die organische Reaktionslösung kann aber auch zur Wassersättigung nach dem Kontakt mit der Hydrolase durch eine wäßrige Phase geleitet oder mit einer wäßrigen Phase vermischt und absetzen gelassen werden. Als wäßrige Phase kommt reines Wasser, eine Pufferlösung oder eine Salzlösung in Betracht. Bevorzugt wird der pH-Wert der wäßrigen Phase, der durch das Einbringen der bei der Reaktion gebildeten Säure sinkt, durch Zugabe einer Base etwa konstant gehalten. Der pH-Wert soll nicht unter 3 absinken und nicht über 11 ansteigen. Bevorzugt wird ein pH-Bereich von 5 bis 10, besonders bevorzugt von 6 bis 8 eingehalten.

Als Basen eignen sich zur Neutralisation übliche Basen, beispielsweise Alkali- oder Erdalkalihydroxide, -carbonate, -hydrogencarbonate, $NH_4OH$ usw., wobei bevorzugt Alkalihydroxide wie Kalium- oder Natriumhydroxid eingesetzt werden. Die Base wird als wäßrige Lösung bevorzugt in Kombination mit einer pH-Wert-Meßanlage, z.B. einer Wasserstoffelektrode, bevorzugt automatisiert, zugegeben. Durch Zugabe der Base bildet die Carbonsäure ein Salz mit der zugegebenen Base, welches in der wäßrigen Phase verbleibt, sodaß die gebildete Carbonsäure dem Hydrolysegleichgewicht entzogen wird, während sich die organische Lösung von der wäßrigen Phase infolge ihrer geringen Mischbarkeit trennt und sich dabei mit Wasser sättigt. Die wassergesättigte organische Lösung wird dann wieder über die Hydrolase geleitet. Diese Verfahrensführung wird solange fortgesetzt, bis der gewünschte Umsetzungsgrad erreicht ist. Die Reaktion wird sodann abgebrochen und die Reaktionsprodukte werden isoliert und gegebenenfalls gereinigt.

Die im Verlauf des Verfahrens gebildete Carbonsäure, die wie oben beschrieben als Salz isoliert wird, kann durch Ansäuern und gegebenenfalls Extraktion wie üblich gewonnen und durch Destillation, Chromatographie oder Umkristallisieren gereinigt werden.

Ist nicht die im Verlauf des Verfahrens entstehende Carbonsäure sondern das zweite Hydrolyseprodukt, also z.B. der Akohol, das Amin, das Thiol oder sind sowohl Carbonsäure als auch der Alkohol, das Amin oder das Thiol das erwünschte Produkt, oder wurde bei der Wassersättigung der organischen Reaktionslösung keine Base zugegeben, werden die erwünschten Produkte nach Abbruch der Reaktion aus der organischen Reaktionslösung auf übliche Art und Weise, beispielsweise durch Extraktion, Destillation, Kristallisation und Umkristallisieren oder Chromatographie erhalten und/oder gereinigt.

Es hat sich unerwarteterweise gezeigt, daß die Enzymaktivität durch Spülen der Hydrolase mit Chloroform nach einem Reaktionszyklus und vor dem folgenden Reaktionszyklus erheblich gesteigert werden kann, falls nicht Chloroform ohnehin als organisches Lösungsmittel für das Carbonsäurederivat eingesetzt wurde. So wurde die spezifische Aktivität einer Lipase innerhalb einiger Reaktionszyklen insgesamt von 138 auf 339 mMol pro Stunde pro Gramm Lipase gesteigert, worauf die Aktivität der Lipase in etwa konstant blieb.

Das Verfahren wird zweckmäßig bei einer Temperatur, bei der die Hydrolase die höchste Aktivität zeigt, durchgeführt. Dabei liegen die Temperaturen im allgemeinen zwischen 0 und 40 °C, bevorzugt zwischen 20 und 30 °C, besonders bevorzugt bei Raumtemperatur, in speziellen Fällen auch höher, aber jedenfalls unter dem Siedepunkt des Lösungsmittels und unter der Deaktivierungstemperatur der verwendeten Hydrolase.

Das Verfahren kann kontinuierlich oder diskontinuierlich durchgeführt werden und wird bevorzugt kontinuierlich durchgeführt.

Ist im Falle der Verwendung chiraler oder prochiraler Carbonsäurederivate der Enantiomerenüberschuß eines Enantiomeren im erhaltenen Produkt, das bei der Reaktion mit der Hydrolase entsteht, infolge einer geringen Stereospezifität der Hydrolase nicht ausreichend, kann das nach einem Reaktionszyklus erhaltene Produkt erneut in ein Carbonsäurederivat übergeführt und in das erfindungsgemäße Verfahren eingesetzt werden, wobei eine weitere Anreicherung des gewünschten Enantiomeren des Produktes erreicht wird. Manchmal ist es auch zur Erreichung einer höheren Enantiomerenreinheit zweckmäßig, das Verfahren schon nach kleinen Umsetzungsraten abzubrechen.

In einer besonderen Ausführungsform des Verfahrens wird ein optisch aktives Carbonsäurederivat, insbesonders ein optisch aktiver Carbonsäureester, der das chirale Zentrum im Säureteil aufweist, wobei das gewünschte Produkt vorwiegend ein Enantiomer einer optisch aktiven Carbonsäure ist, in Diisopropylether oder Toluol möglichst konzentriert gelöst und in einem Vorratsbehälter, in dem eine wäßrige Phase vorgelegt wurde unter Rühren durch Mischen mit der wäßrigen Phase mit Wasser gesättigt. Die wassergesättigte organische Phase wird mit Hilfe einer Pumpe aus dem Vorratsbehälter über eine Lipase geleitet, die adsorbiert an einen inerten Träger außerhalb des Vorratsbehälters zweckmäßigerweise in eine Säule eingebracht ist, sodaß die Lipase nicht mit der wäßrigen Phase des Vorratsbehälters in Kontakt kommt. Dabei findet die Hydrolyse in enzymspezifischem Ausmaß statt. Die organische Reaktionslösung wird dann in die wäßrige Phase des Vorratsbehälters zurückgeleitet. Der pH-Wert der wäßrigen Phase, der durch das Einbringen der gebildeten Carbonsäure absinkt, wird durch Zugabe einer wäßrigen Natriumhydroxid- oder Kaliumhydroxidlösung etwa auf pH 7 gehalten. Dadurch bildet die Carbonsäure ein Salz und verbleibt in der wäßrigen Lösung. Der freigesetzte Alkohol und die nicht umgesetzte Ausgangsverbindung verbleiben gelöst in der organischen Reaktionslösung, die sich infolge ihrer geringen Mischbarkeit mit Wasser von der wäßrigen Phase des Vorratsbehälters trennt und in einem Kreisprozess so lange wieder über die Hydrolase und dann wieder über die wäßrige Phase geleitet wird, bis der gewünschte Umsetzungsgrad erreicht ist. Die Reaktion wird sodann abgebrochen und die wäßrige Phase von der organischen getrennt. Zur Isolierung der gebildeten Carbonsäure wird die wäßrige Phase wie üblich angesäuert und die freigesetzte Carbonsäure extrahiert. Eine Reinigung durch Umkristallisieren, Destillation oder Chromatographie kann angeschlossen werden, wobei durchaus eine weitere Anreicherung des gewünschten Enantiomeren erreicht werden kann.

In einer besonders bevorzugten Ausführungsform des erfindungsgemäßen Verfahrens wird destilliertes Wasser in einem Vorratsbehälter vorgelegt und unter Rühren mit der Lösung eines enantiomeren Gemisches eines Carbonsäureesters, der in 2-Stellung des Säureteils, bevorzugt durch Halogen substituiert ist, in Diisopropylether versetzt und absetzen gelassen. Die organische Phase wird daraufhin kontinuierlich über eine Lipase, die sich an Celite adsorbiert außerhalb des Voratsbehälters, in eine Säule gepackt, befindet und danach in die wäßrige Phase des Vorratsbehälters zurückgeleitet, wobei der pH-Wert der wäßrigen Phase durch Zugabe eines wäßrigen Alkalihydroxids in einem Bereich von 6 bis 9 konstant gehalten wird. Die wäßrige Phase kann dabei dem Vorratsbehälter entnommen und durch Zugabe frischer, wäßriger Phase ersetzt werden. Auch die organische Phase, die vorwiegend das von Enzym nicht verwertete Enantiomere des chiralen Carbonsäureesters und den gebildeten Alkohol enthält, kann nach Erreichen des gewünschten Umsetzungsgrades dem Vorratsbehälter entnommen und durch frische organische Phase ersetzt werden. Die Gewinnung des reinen oder angereicherten Enantiomeren der optisch aktiven Carbonsäure aus der wäßrigen Lösung erfolgt durch Ansäuern und Extraktion der wäßriger Lösung mit einem organischen Lösungsmittel für die Carbonsäure.

Mit Hilfe des erfindungsgemäßen Verfahrens wird auf einfache Weise ein Carbonsäurederivat in einem organischen Lösungsmittel enzymatisch hydrolisiert und das erwünschte Produkt auf einfache Weise isoliert, wobei das Enzym nicht immobilisiert vorgelegt werden muß, wobei insbesonders ein Enantiomerengemisch einer chiralen Ausgangsverbindung gegebenenfalls durch Anwendung mehrerer Reaktionszyklen in ein hochangereichertes Enantiomeres einer optisch aktiven Verbindung übergeführt werden kann, wobei das Lösungsmittel immer wieder verwendet wird, wobei praktisch kein Enzymverlust oder Aktivitätsverlust des Enzyms über lange Zeiträume auftritt und wobei keine umweltschädigenden Abfallprodukte entstehen und wiederaufbereitet oder entsorgt werden müssen. Das Verfahren stellt somit eine Bereicherung der Technik dar.

Beispiel 1

20,05 g eines Enantiomerengemisches aus 5,61 g S- und 14,44 g R-2-Brompropionsäure-2-ethyl-hexylester, das entspricht einem Enantiomerenüberschuß des R-Enantiomeren von 44 %, hergestellt durch Veresterung eines entsprechenden Enantiomerengemisches von 2-Brompropionsäure mit 2-Ethylhexanol, wurden mit Diisopropylether gelöst, sodaß 170 ml Lösung entstanden. Die Lösung wurde in 140 ml Wasser, das mit 5,5 ml Ethanol versetzt und in einem Behälter vorgelegt worden war, eingetragen und verrührt.

Dabei bildeten sich 2 Phasen. Die wassergesättigte organische Phase wurde anschließend über 1,5 g einer Candida cylindracea Lipase, die vermischt mit 12 g Celite außerhalb des Behälters in eine Säule gepackt angebracht war, mit einer ungefähren Pumpgeschwindigkeit von 100 ml/min gepumpt. Nach dem Durchgang durch die Säule wurde die organische Reaktionslösung in die wäßrige Phase des Behälters geleitet. Der pH-Wert der wäßrigen Phase wurde automatisch durch Zugabe von wäßrigem 2 M Natriumhydroxid auf 5 bis 8 gehalten. Dadurch bildete sich das Natriumsalz der bei der Reaktion entstandenen 2-Brompropionsäure, das in der wäßrigen Lösung verblieb, während sich die organische Reaktionslösung mit Wasser sättigte und von der wäßrigen Phase des Behälters trennte. Die nunmehr wieder wassergesättigte organische Lösung wurde wieder kontinuierlich über die Lipase und anschließend durch die wäßrige Phase gepumpt. Nach 0,5 Stunden waren dabei 2,05 ml, nach 1,5 Stunden 5,85 ml 2 M wäßriges Natriumhydroxid verbraucht und ein Umsetzungsgrad von 15 % erreicht. Die wäßrige Lösung wurde anschließend mit Schwefelsäure angesäuert, die dadurch freigesetzte 2-Brompropionsäure mit Hilfe von Diisopropylether extrahiert und durch Abdampfen des Extraktionsmittels isoliert. Dabei wurden 1,65 g eines Enantiomerengemisches von R- und S-2-Brompropionsäure mit einem optischen Drehwert $(alpha)_D^{20}$ von $+25,4°$ das entspricht einen Enantiomerenüberschuß von 90,4 % des R-Enantiomeren erhalten, d.h. es waren 0,08 g S- und 1,57 g R-2-Brompropionsäure gebildet worden. Die spezifische Aktivität der Lipase betrug 5,2 mMol pro Stunde pro Gramm Lipase.

Beispiele 2 bis 10

Die Beispiele 2 bis 7 wurden auf die gleiche Art und Weise wie im Beispiel 1 beschrieben, unter Verwendung derselben Candida cylindracea Lipase des Beispiels 1 ausgeführt, wobei die Reaktionszeit auf etwa 4 Stunden ausgedehnt und bei Erreichen eines Umsetzungsgrades von etwa 44 % abgebrochen wurde. Die Säule mit der Candida cylindracea Lipase wurde nach jedem Reaktionszyklus mit Diisopropylether gespült.

Die Beispiele 8 bis 10 wurden auf die gleiche Art und Weise, wie im Beispiel 1 beschrieben, jedoch unter Verwendung der 6-fachen Menge an Enantiomerengemisch des 2-Brompropionsäure-2-ethylhexylesters, an Diisopropylether, an Wasser und an Ethanol unter Verwendung derselben Candida cylindracea Lipase der Beispiele 1 bis 7 ausgeführt. Die Säule mit der Candida cylindracea Lipase wurde nach jedem Reaktionszyklus mit Diisopropylether gespült.

Dabei wurden die Ergebnisse, die in den Tabellen 1 und 2 zusammengefaßt sind, erhalten.

Tabelle 1

| h | Beispiele | | | | | | | | |
|---|---|---|---|---|---|---|---|---|---|
| | 2 | 3 | 4 | 5 | 6 | 7 | 8 | 9 | 10 |
| | Prozent Umsetzung nach Stunden | | | | | | | | |
| 0,5 | 7,3 | | 6,3 | | 7,1 | 5,8 | 3,3 | 2,2 | 1,8 |
| 1,0 | 13,8 | | | | 14,6 | | | | |
| 1,5 | | 19,6 | 20,4 | 20,5 | | | | | |
| 2,0 | | 24,9 | | 26,2 | 29,1 | | | | |
| 2,5 | 29,9 | 29,9 | 32,0 | 30,4 | 34,9 | 27,5 | 7,3 | 5,3 | |
| 3,0 | | 34,7 | | | 39,4 | | | | |
| 3,5 | 38,9 | | | | | | | | |
| 4,0 | 42,6 | | 46,6 | | | | | | |
| 4,2 | 43,9 | 43,9 | | 44,2 | 43,9 | | | | |
| 4,5 | | | | | | 43,9 | | | |
| 18,0 | | | | | | | | | 35,7 |
| 19,0 | | | | | | | | 37,7 | |
| 20,5 | | | | | | | 39,4 | | |
| 24,13 | | | | | | | 44,1 | | |
| 24,25 | | | | | | | | 44,1 | 44,1 |

Tabelle 2

| Beispiel | G (g) | alpha(°) | ee(%) | Akt |
|---|---|---|---|---|
| 2 | 4,66 | +27,1 | 96,4 | 5,3 |
| 3 | 4,67 | +26,2 | 93,2 | 5,3 |
| 4 | 4,91 | +26,4 | 94,0 | 5,9 |
| 5 | 4,66 | +28,2 | 100,4 | 5,3 |
| 6 | 4,68 | +25,7 | 91,5 | 5,4 |
| 7 | 4,60 | +28,6 | 101,3 | 4,9 |
| 8 | 28,0 | +27,3 | 97,2 | 5,5 |
| 9 | 27,7 | +27,7 | 98,6 | 5,5 |
| 10 | 28,03 | +28,5 | 101,0 | 5,5 |

Die Candida cylindracea Lipase des Beispieles 1 wurde also insgesamt etwa 95 Stunden eingesetzt, ohne an Aktivität zu verlieren. In den Tabellen bedeutet

| | |
|---|---|
| h: | Reaktionszeit (Stunden) |
| Prozent Umsetzung: | Umgesetzter Anteil des Enantiomerengemisches des 2-Brompropionsäure-2-ethyl-hexylesters |
| G(g): | Ausbeute in Gramm |
| alpha(°): | optischer Drehwert $(alpha)_D^{20}$ |
| ee(%): | Enantiomerenüberschuß der gewonnenen R-gegenüber der S-2-Brompropionsäure |
| Akt: | spezifische Aktivität der Candida cylindracea Lipase in mMol pro Stunde pro Gramm Lipase |

Beispiel 11

20 g einer racemischen Mischung von R- und S-2-Brompropionsäure-2-ethyl-hexylester wurden in 150 ml Diisopropylether gelöst. Diese Lösung wurde in einem Behälter, in dem 70 ml Wasser vorgelegt worden waren, eingetragen und verrührt. Dabei bildeten sich 2 Phasen. Die organische wassergesättigte Phase wurde anschließend über 50 mg einer Candida cylindracea Lipase, vermischt mit 2,8 g Celite, eingebracht in eine Säule außerhalb des Behälters mit einer ungefähren Pumpgeschwindigkeit von 100 ml/min gepumpt. Nach dem Durchgang durch die Säule wurde die organische Reaktionslösung wie im Beispiel 1 beschrieben, behandelt.

Nach 6,22 Stunden war ein Umsetzungsgrad von 29,17 % erreicht und die Reaktion wurde abgebrochen. Dabei wurden 4,3 g eines Enantiomerengemisches von R- und S-2-Brompropionsäure mit einem optischen Drehwert $(alpha)_D^{20}$ von +20,6°, das entspricht einem Enantiomerenüberschuß des R-Enantiomeren von 73,3 %, erhalten.

Die spezifische Aktivität der Lipase betrug 70,78 mMol pro Stunde pro Gramm Lipase.

Beispiele 12 - 17

Wurden wie im Beispiel 11 beschrieben unter Verwendung der gleichen Ausgangsmengen durchgeführt, wobei die Säule, die dieselbe Lipase des Beispiels 11 enthielt, vor jedem neuen Durchgang mit Chloroform gespült worden war.

Dabei wurden die Ergebnisse erhalten, die in Tabelle 3 zusammengestellt sind.

Tabelle 3

| Beispiel | Prozent Umsetzung | nach Zeit (in Stunden) | alpha(°) | ee(%) | Akt. |
|---|---|---|---|---|---|
| 12 | 29,8 | 3,25 | +21,6 | 76,9 | 138,5 |
| 13 | 30 | 1,75 | +22,1 | 78,6 | 258,3 |
| 14 | 29,8 | 2,23 | +21,3 | 75,8 | 236,8 |
| 15 | 30 | 1,58 | +22,7 | 80,8 | 285,5 |
| 16 | 30 | 1,33 | +20,0 | 71,2 | 339,0 |
| 17 | 30 | 1,80 | +17,2 | 61,2 | 251,1 |

Beispiel 18

Wurde wie im Beispiel 11 beschrieben durchgeführt unter Verwendung von 45,1 g eines racemischen Gemisches von R- und S-2-Brompropionsäure-2-ethyl-hexylester und 50 mg einer Candida cylindracea Lipase. Dabei wurde nach 19,25 Stunden ein Umsetzungsgrad von 45 % erzielt. Es wurden 9,88 g eines Enantiomerengemisches von R- und S-2-Brompropionsäure mit einem optischen Drehwert (alpha)$_D^{20}$ von +21,5°, das entspricht einem Enantiomerenüberschuß des R-Enantiomeren von 76,5 %, erhalten.

Beispiel 19

Die in Beispiel 18 erhaltene 2-Brompropionsäure wurde mit 2-Ethylhexanol chemisch verestert. Der Ester wurde wie im Beispiel 18 beschrieben mit einer dort beschriebenen Lipase umgesetzt. Dabei wurde nach 15,25 Stunden ein Umsetzungsgrad von 72,3 % erzielt. Dabei wurden 6,62 g eines Enantiomerengemisches von R- und S-2-Brompropionsäure mit einem optischen Drehwert (alpha)$_D^{20}$ von +26,4°, das entspricht einem Enantiomerenüberschuß der R-2-Brompropionsäure von 94 %, erhalten.

Beispiel 20 bis 25

Die Beispiele 20 - 21, 22 - 23 und 24 - 25 wurden wie die Beispiele 18 - 19 unter Verwendung jeweils derselben Menge an Ausgangsstoffen und unter jeweiliger Verwendung derselben Candida cylindracea Lipase des Beispiels 18 durchgeführt. Die Ergebnisse sind in Tabelle 4 zusammengefaßt.

Tabelle 4

| Bei spiel | Umsetzung (%) | nach Zeit (h) | Ausbeute (g) | alpha (°) | ee(%) |
|---|---|---|---|---|---|
| 20 | 53,6 | 17 | 12,6 | +18,7 | 66,5 |
| 21 | 63,7 | 22 | 8,0 | +26,2 | 93,2 |
| 22 | 42,6 | 22 | 11,1 | +17,8 | 63,3 |
| 23 | 62,9 | 26,75 | 6,7 | +26,2 | 93,2 |
| 24 | 43,2 | 25,5 | 11,5 | +15,9 | 56,6 |
| 25 | 57,2 | 19,5 | 6,5 | +27,2 | 96,8 |

Beispiel 26

41 g eines racemischen Gemisches von R- und S-2-Chlorpropionsäurebutylester wurden in 360 ml Diisopropylether gelöst und mit 200 ml Wasser, das in einem Behälter vorgelegt wurde, unter Rühren eingebracht. Dabei bildeten sich 2 Phasen. Die organische Oberphase wurde über 15 g einer Geotrichum candidum Lipase, die mit 60 g Celite vermischt in einer Säule außerhalb des Behälters angebracht war, mit einer Pumpgeschwindigkeit von 100 ml/min gepumpt. Die organische Reaktionslösung wurde danach in die wäßrige Phase des Behälters zurückgeleitet, die mit Hilfe einer automatisierten pH-Meßeinrichtung unter Zugabe von wäßrigen 2 M Natriumhydroxid auf einen pH-Wert zwischen 6 und 8 gehalten wurde. Dadurch bildete sich das Natriumsalz der bei der Reaktion entstandenen 2-Chlorpropionsäure, das in der wäßrigen Phase verblieb. Die organische Reaktionslösung trennte sich von der wäßrigen Phase und wurde kontinuierlich über die Lipase und dann wieder durch die wäßrige Phase gepumpt.

Nach 44,5 Stunden war ein Umsetzungsgrad von 20,6 % erreicht und die Reaktion wurde abgebrochen. Die wäßrige Lösung wurde durch Zugabe von Schwefelsäure angesäuert und mit Chloroform extrahiert und das organische Lösungsmittel über Natriumsulfat getrocknet und abgedampft.

Dabei wurden 5,3 g eines Enantiomerengemisches von R- und S-2-Chlorproprionsäuremit einem optischen Drehwert (alpha)$_D^{20}$ von -12°, das entspricht einem Enantiomerenüberschuß des S-Enantiomeren von 73,2 %, erhalten.

Beispiel 27

55,5 g eines racemischen Gemisches von R- und S-2-Chlorpropionsäure-2-ethyl-hexylester wurden in 360 ml Diisopropylether gelöst und in einen Behälter, in dem 200 ml Wasser vorgelegt waren, unter Rühren eingebracht. Dabei bildeten sich 2 Phasen. Die organische, wassergesättigte Oberphase wurde über 1 g einer Candida cylindracea Lipase, die sich vermischt mit 10 g Celite in eine Säule gepackt außerhalb des Behälters befand, geleitet. Anschließend wurde wie im Beispiel 1 beschrieben verfahren. Nach Erreichen eines Umsetzungsgrades von 32 % wurde die Reaktion abgebrochen. Dabei wurde ein Enantiomergemisch von R- und S-2-Chlorpropionsäure mit einem optischen Drehwert (alpha)$_D^{20}$ von +6,9°, das entspricht einem Enantiomerenüberschuß des R-Enantiomeren von 42,1 % erhalten. Die spezifische Aktivität der Lipase betrug 11,58 mMol pro Stunde pro Gramm Lipase.

Beispiel 28

55,5 g eines racemischen Gemisches von R- und S-2-Chlorpropionsäure-2-ethyl-hexylester wurden in 360 ml Diisopropylether gelöst und in einem Behälter, in dem 200 ml Wasser vorgelegt waren, unter Rühren eingebracht. Dabei bildeten sich 2 Phasen. Die organische, wassergesättigte Oberphase wurde mit einer Pumpgeschwindigkeit von 100 ml/min über eine Säule, die 2 g einer Candida cylindracea Lipase, die durch Rühren mit 2 ml 50 % Glutardialdehyd in Diisopropylether vernetzt und mit 13 g Celite vermischt worden war, enthielt, gepumpt. Anschließend wurde wie im Beispiel 1 beschrieben verfahren. Nach Erreichen eines Umsetzungsgrades von 32 % wurde die Reaktion abgebrochen. Dabei wurde ein Enantiomerengemisch von R- und S-2-Chlorpropionsäure mit einem Drehwert (alpha)$_D^{20}$ von +7,3°, das entspricht einem Enantiomerenüberschuß des R-Enantiomeren von 44,5 %, erhalten. Die spezifische Aktivität der Lipase betrug 4,8 mMol pro Stunde pro Gramm Lipase.

Beispiel 29

Unter Verwendung von 55,2 g eines racemischen Enantiomerengemisches von 2-Chlorpropionsäure-2-ethyl-hexylester, 400 ml Hexan anstatt Diisopropylether und 3 g einer Candida cylindracea Lipase, vermischt mit 21 g Celite wurden nach der im Beispiel 26 beschriebenen Art und Weise nach 19 Stunden ein Umsetzungsgrad von 78,6 % erreicht. Dabei wurde ein Enantiomerengemisch von R- und S-2-Chlorpropionsäure mit einem optischen Drehwert (alpha)$_D^{20}$ von +2,5°, das entspricht einem Enantiomerüberschuß des R-Enantiomeren von 15,2 %, erhalten. Der Enantiomerenüberschuß des S-Enantiomeren im nicht umgesetzten Ester betrug 56 %.

Beispiel 30

Unter Verwendung von 294,1 g eines racemischen Enantiomerengemisches von 2-Chlorpropionsäure-2-ethyl-hexylester, 100 ml Diisopropylether und 20 ml Aceton anstatt reinem Diisopropylether und 10 g Candida cylindracea Lipase, vermischt mit 50 g Celite wurde nach der im Beispiel 26 beschriebenen Art und Weise bis zu einem Umsetzungsgrad von 57 % verfahren. Dabei wurde ein Enantiomerengemisch von R- und S-2-Chlorpropionsäure mit einem optischen Drehwert (alpha)$_D^{20}$ von +6,1°, das entspricht einem Enantiomerenüberschuß des R-Enantiomeren von 37 %, erhalten.

Beispiel 31

Unter Verwendung von 57,2 g eines racemischen Enantiomerengemisches von R- und S-2-Chlorpropionsäurephenylethylester, gelöst in 400 ml Diisopropylether und 3 g einer Candida cylindracea Lipase, vermischt mit 15 g Celite, wurde nach der im Beispiel 26 beschriebenen Art und Weise nach 1,30 Stunden ein Umsetzungsgrad von 60 % erreicht. Dabei wurde ein Enantiomerengemisch von R- und S-2-Chlorpropionsäure mit einem optischen Drehwert (alpha)$_D^{20}$ von +6,1°, das entspricht einem Enantiomerenüberschuß des R-Enantiomeren von 37 %, erhalten.
Die spezifische Aktivität der Lipase betrug 41,7 mMol pro Stunde pro Gramm Lipase.

Beispiel 32

Wurde wie Beispiel 31 unter Verwendung von 159 g eines racemischen Enantiomerengemisches von R- und S-2-Chlorpropionsäurephenylethylester als Ausgangsmaterial, gelöst in 400 ml Diisopropylether und derselben im Beispiel 31 verwendeten Lipase, die mit Diisopropylether gespült worden war, ausgeführt. Nach 4,12 Stunden war ein Umsetzungsgrad von 67 % erreicht und die Reaktion wurde abgebrochen. Dabei wurde ein Enantiomerengemisch von R- und S-2-Chlorpropionsäure mit einem optischen Drehwert (alpha)$_D^{20}$ von +5,3°, das entspricht einem Enantiomerenüberschuß des R-Enantiomeren von 32,3 %, erhalten.
Die spezifische Aktivität der Lipase betrug 40,8 mMol pro Stunde pro Gramm Lipase.

Beispiel 33 bis 35

41 g eines racemischen Enantiomerengemisches von 2-Chlorpropionsäurebutylester wurden in 360 ml Lösungsmittel gelöst und in einem Behälter, in dem 200 ml Wasser vorgelegt worden waren, unter Rühren eingebracht. Dabei bildeten sich 2 Phasen. Die organische, wassergesättigte Phase wurde über einer Säule, die 3 g einer Candida cylindracea Lipase, vermischt mit 9 g Celite, enthielt, bis zu einem Umsetzungsgrad von 33 % gepumpt. Anschließend wurde, wie in Beispiel 1 beschrieben, verfahren. Dabei wurden folgende Ergebnisse erhalten:

| Beispiel | Lösungsmittel | Akt | alpha |
|---|---|---|---|
| 33 | Diisopropylether | 27,18 | +5,4 |
| 34 | n-Heptan | 12,78 | +5,2 |
| 35 | Chloroform | 2,17 | +5,6 |

Akt: spezifische Aktivität in mMol pro Stunde pro Gramm Lipase
alpha: optischer Drehwert (alpha)$_D^{20}$

Beispiel 36

55,2 g eines racemischen Gemisches von 2-Chlorpropionsäure-2-ethyl-hexylesters wurden in 400 ml Diisopropylether gelöst und in einem Behälter, in dem 200 ml Wasser vorgelegt worden waren, unter Rühren eingebracht. Dabei bildeten sich 2 Phasen. Die organische, wassergesättigte Phase wurde über 6 g einer Humicola lanuginosa Lipase, vermischt mit 20 g Celite geleitet. Anschließend wurde, wie in Beispiel 1 beschrieben, verfahren. Nach 25 Stunden war ein Umsetzungsgrad von 32,4 % erreicht und die Reaktion wurde abgebrochen.
Dabei wurde ein Enantiomerengemisch von R- und S-2-Chlorpropionsäure mit einem optischen Drehwert (alpha)$_D^{20}$ von +8,9°, das entspricht einem Enantiomerenüberschuß des R-Enantiomeren von 54,3 %, erhalten. Die spezifische Aktivität der Lipase betrug 0,54 mMol pro Stunde pro Gramm Lipase.

Beispiel 37

wurde wie Beispiel 36 unter Verwendung der gleichen Ausgangsprodukte und -mengen und unter Verwendung derselben, in Beispiel 36 eingesetzten Lipase nach Spülen der enzymenthaltenden Säule mit Diisopropylether wiederholt. Nach 48 Stunden war ein Umsetzungsgrad von 64,8 % erreicht und die Reaktion wurde abgebrochen. Dabei wurde ein Enantiomerengemisch von R- und S-2-Chlorpropionsäure mit einem optischen Drehwert (alpha)$_D^{20}$ von +4,7°, das entspricht einem Enantiomerenüberschuß des R-Enantiomeren von 28,7 %, erhalten. Die spezifische Aktivität der Lipase betrug 0,56 mMol pro Stunde pro Gramm Lipase.

Beispiel 38

Unter Verwendung der organischen Phase aus Beispiel 37, die 19,4 g 2-Chlorpropionsäure-2-ethyl-hexylester enthielt, und einer Pseudomonas fluorescens Lipase, die mit 12,5 g Celite vermischt war, wurde auf die im Beispiel 36 beschriebene Art und Weise nach 8,6 Stunden ein Umsatz von 35,8 % erreicht. Dabei wurde ein Enantiomerengemisch von R- und S-2-Chlorpropionsäure mit einem Drehwert (alpha)$_D^{20}$ von -12,6°, das entspricht einem Enantiomerenüberschuß des S-Enantiomeren von 76,8 %, erhalten. Die Aktivität der Lipase betrug 1,48 mMol pro Stunde pro Gramm Lipase.

Beispiel 39

Unter Verwendung von 102,5 g eines Enantiomerengemisches von R- und S-2-Chlorpropionsäure-2-ethyl-hexylester, mit einem Enantiomerenüberschuß des S-Enantiomeren von 58,5 %, hergestellt durch Veresterung der entsprechenden 2-Chlorpropionsäureenantiomeren mit 2-Ethyl-hexanol, und 4,8 mg einer Chromabakterium viscosum Lipase, vermischt mit 10 g Celite, wurde auf die im Beispiel 36 beschriebene Art und Weise ein Umsetzungsgrad von 22,5 % erreicht. Dabei wurden 4,7 g eines Enantiomerengemisches von R- und S-2-Chlorpropionsäure mit einem optischen Drehwert (alpha)$_D^{20}$ von -12,9°, das entspricht einem Enantiomerenüberschuß des S-Enantiomeren von 78,7 %, erhalten. Die spezifische Aktivität der Lipase betrug 1,04 Mol pro Stunde pro Gramm Lipase.

Beispiel 40

57,6 g eines in Beispiel 27 beschriebenen racemischen Enantiomerengemisches wurde auf die im Beispiel 27 beschriebene Art und Weise aber bei 0 ±1,5 °C mit 6 g einer Candida cylindracea Lipase, die mit 24 g Celite vermischt war, in 5 Stunden mit einem Umsetzungsgrad von 70 % zu einem Enantiomerengemisch aus R- und S-2-Chlorpropionsäure mit einem optischen Drehwert (alpha)$_D^{20}$ von +4,3°, das entspricht einem Enantiomerenüberschuß von 26 %, umgesetzt. Die spezifische Aktivität der Lipase betrug 5,83 mMol pro Stunde pro Gramm Lipase.

Beispiel 41

Auf die im Beispiel 40 beschriebene Art und Weise, aber bei einer Temperatur von 10 °C wurde in 2,8 Stunden ein Umsetzungsgrad von 70 % erreicht. Die spezifische Aktivität der Lipase betrug 10,3 mMol pro Stunde pro Gramm Lipase. Der optische Drehwert (alpha)$_D^{20}$ des entstandenen Enantiomerengemisches betrug 4,4°.

Beispiel 42

56,6 g eines racemischen Enantiomerengemisches von 2-Chlorpropionsäure-2-ethyl-hexylester wurden in 400 ml wassergesättigtem Diisopropylether gelöst und in einem Behälter vorgelegt. Die Lösung wurde über 3 g einer Candida cylindracea Lipase, die mit 15 g Celite vermischt war, gepumpt. Die dabei gebildete Reaktionslösung wurde anschließend über eine Säule gepumpt, die 10 g Kalciumhydroxid, vermischt mit 20 g Celite, enthielt. Dabei verblieb die bei der Reaktion gebildete 2-Chlorpropionsäure infolge von Salzbildung in der Säule, während das bei der Hydrolyse verbrauchte Wasser in der organischen Lösung wieder ersetzt wurde. Die weitere Reaktionsführung erfolgte kontinuierlich auf die oben beschriebene Art und Weise, bis nach 4 Stunden ein Umsetzungsgrad von 34,3 % erreicht war. Dabei wurde ein Enantiomerengemisch der R- und S-2-Chlorpropionsäure mit einem optischen Drehwert (alpha)$_D^{20}$ von +7,0°, das entspricht einem Enantiomerenüberschuß des R-Enantiomeren von 43 %, erhalten.

Beispiel 43

6,59 g eines racemischen Enantiomerengemisches von 2-Chlorpropionsäurebutylester wurden in 75 ml Diisopropylether gelöst und mit 1,6 ml eines Natriumphosphatpuffers (pH = 7), 0,1 g Sephadex G 50 der Fa. Pharmacia, vorgequollen in Wasser, 6 g Celite und 6 g einer Geotrichum candidum Lipase versetzt und bei Raumtemperatur gerührt. Nach 168,8 Stunden war ein Umsetzungsgrad von 37,4 % erreicht und die Reaktion wurde abgebrochen. Dabei wurde ein Enantiomerengemisch von R- und S-2-Chlorpropionsäure mit einem optischen Drehwert von (alpha)$_D^{20}$ von -8,8°, das entspricht einem Enantiomerenüberschuß des S-Enantiomeren von 53,7 %, erhalten.

Beispiel 44

Wie im Beispiel 43 beschrieben, aber unter Verwendung von 15 g eines racemischen Enantiomerengemisches von 2-Chlorpropionsäurepropylester, 5 g einer Geotrichum candidum Lipase, 5 g Celite, 2,5 ml eines 10 mM Natriumphosphatpuffers (pH = 7), 340 mg Hydrogel Evergreen 500, Chemie Linz AG, vorgequollen in Wasser, wurde nach 118,5 Stunden ein Umsatz von 24,5 % erreicht. Dabei wurde ein Enantiomerengemisch der R- und S-2-Chlorpropionsäure mit einem optischen Drehwert $(alpha)_D^{20}$ von +3,0°, das entspricht einem Enantiomerenüberschuß des R-Enantiomeren von 18,3 %, erhalten.

Beispiel 45

Auf die im Beispiel 43 beschriebene Art und Weise aber unter Verwendung von 0,1 g Hydrogel Evegreen 500, Chemie Linz AG, vorgequollen in Wasser, anstatt Sephadex G 50, wurde nach 46,8 Stunden ein Umsetzungsgrad von 24 % erreicht. Dabei wurde ein Enantiomerengemisch von R- und S-2-Chlorpropionsäure mit einem optischen Drehwert $(alpha)_D^{20}$ von -11,7°, das entspricht einem Enantiomerenüberschuß des S-Enantiomeren von 71,3 %, erhalten.

Beispiel 46

6,58 g eines racemischen Enantiomerengemisches von 2-Chlorpropionsäurebutylester wurden in 50 ml Diisopropylether gelöst und mit 1 g Celite, 0,1 g Sephadex G 50 der Firma Pharmacia, vorgequollen in Wasser, 0,8 g 10 mM Natriumphosphatpuffer (pH = 7) und 0,8 g einer Candida cylindracea Lipase versetzt und bei Raumtemperatur gerührt. Nach 1,75 Stunden betrug der Umsetzungsgrad 34,5 %. Die Reaktion wurde abgebrochen und das Hydrogel und die Lipase abfiltriert. Die Reaktionslösung enthielt ein Enantiomerengemisch von R- und S-2-Chlorpropionsäure mit einem Drehwert $(alpha)_D^{20}$ von +4,3°, das entspricht einem Enantiomerenüberschuß des R-Enantiomeren von 26,2 %. Die spezifische Aktivität der Lipase betrug 9,86 mMol pro Stunde pro Gramm Lipase.

Der in den Beispielen angegebene spezifische, optische Drehwert der erhaltenen Produkte $(alpha)_D^{20}$ wurde in allen Beispielen bei einer Wellenlänge von 589 nm (Natrium D-Linie), 20 °C, c = 1 in Chloroform gemessen.

Als Celite wurde in den Beispielen "Celite Hyflo Super-Cel" der Fa. Fluka, Partikelgröße 2 bis 25 µ eingesetzt.

Beispiel 47 - 52

Jeweils 7,0 g eines racemischen Enantiomerengemisches von R- und S-Octansäure-1-phenylethylester wurden in 300 ml Diisopropylether gelöst und in einem Behälter, in dem 200 ml Wasser vorgelegt worden waren, unter Rühren eingebracht. Dabei bildeten sich zwei Phasen. Die organische, wassergesättigte Phase wurde, wie in Beispiel 1 beschrieben, über eine Säule, die 3 g Candida cylindracea Lipase der Firma Meito, vermischt mit 22 g Celite, enthielt, gepumpt, wobei dieselbe Lipase für alle Beispiele 47 bis 52 verwendet wurde. Der pH-Wert der wäßrigen Phase wurde, wie in Beispiel 1 beschrieben, auf 5 bis 8 gehalten. Nach Erreichen eines Umsetzungsgrades von etwa 30 % wurde die wäßrige Phase mit Diisopropylether extrahiert und die Reaktion abgebrochen. Nach Abdampfen des Diisopropylethers wurde aus dem verbliebenen Rückstand 1-Phenylethanol mit Hilfe von Vakuumdestillation isoliert. Dabei wurden die in Tabelle 5 zusammengefaßten Ergebnisse erhalten.

Tabelle 5

| Beispiel | Prozent Umsetzung | nach Zeit (in Stunden) | alpha (°) | ee (%) | Akt |
|---|---|---|---|---|---|
| 47 | 32 | 22,0 | 41,2 | 92 | 0,137 |
| 48 | 30 | 22,0 | - | - | 0,128 |
| 49 | 31 | 23,0 | 41,2 | 92 | 0,127 |
| 50 | 26 | 20,5 | - | - | 0,119 |
| 51 | 29 | 24,5 | - | - | 0,111 |
| 52 | 29 | 26,5 | 39,7 | 88 | 0,103 |

Akt: spezifische Aktivität der Candida cylindracea Lipase in mMol pro Stunde pro Gramm Lipase alpha: optischer Drehwert, gemessen bei einer Wellenlänge von 589 nm, 20 °C, c = 1 in Methanol ee Enantiomerenüberschuß des gewonnenen R- gegenüber dem S-1-Phenylethanol - Die Reaktionslösung wurde nicht aufgearbeitet

**Patentansprüche**

1. Verfahren zur enzymatischen Hydrolyse eines Carbonsäurederivates, dadurch gekennzeichnet, daß das Carbonsäurederivat in einem organischen Lösungsmittel, das mit Wasser nur in einem geringen Ausmaß mischbar ist, gelöst wird, worauf die Lösung mit Wasser gesättigt und mit einer Hydrolase in Kontakt gebracht wird, wobei eine Hydrolyse unter Wasserverbrauch stattfindet, worauf die organische Reaktionslösung solange erneut mit Wasser gesättigt und mit der Hydrolase in Kontakt gebracht wird, bis der gewünschte Umsetzungsgrad erreicht ist.

2. Verfahren nach Anspruch 1, dadurch gekennzeichnet, daß die Wassersättigung der organischen Phase mit Hilfe eines wasserenthaltenden Hydrogels erfolgt.

3. Verfahren nach Anspruch 1, dadurch gekennzeichnet, daß die Wassersättigung nach der Hydrolyse durch überleiten der organischen Reaktionslösung über Hydroxidionen enthaltende Agentien erfolgt, wobei die gebildete Carbonsäure dem Reaktionsgleichgewicht entzogen und das bei der Hydrolyse verbrauchte Wasser ersetzt wird.

4. Verfahren nach Anspruch 1, dadurch gekennzeichnet, daß das Carbonsäurederivat im organischen Lösungsmittel gelöst und die gebildete organische Lösung in eine wäßrige Phase eines Vorratsbehälters eingebracht wird, wobei sich die organische Lösung mit Wasser sättigt und sich von der wäßrigen Phase trennt, worauf diese wassergesättigte organische Lösung durch ein Reaktionsgefäß, das die Hydrolase enthält, gepumpt und die gebildete organische Reaktionslösung in die wäßrige Phase des Vorratsbehälters, dessen pH-Wert durch Zugabe einer Base konstant gehalten wird, zurückgepumpt wird, wobei die gebildete Carbonsäure als Salz der zugegebenen Base in der wäßrigen Phase verbleibt und dem Hydrolysegleichgewicht entzogen wird, während sich die organische Phase wieder mit Wasser sättigt, sich vom Wasser trennt und daraufhin solange über die Hydrolase und anschließend in die wäßrige Phase gepumpt wird, bis der gewünschte Umsetzungsgrad erreicht ist.

5. Verfahren nach Anspruch 4, dadurch gekennzeichnet, daß die Hydrolase nach Beendigung eines Reaktionszyklus mit Chloroform gespült wird, bevor sie in einem neuen Reaktionszyklus eingesetzt wird.

6. Verfahren nach einem der Ansprüche 1 bis 5, dadurch gekennzeichnet, daß ein chirales oder prochirales Carbonsäurederivat eingesetzt wird.

7. Verfahren nach einem der Ansprüche 1 bis 6, dadurch gekennzeichnet, daß ein Enantiomerengemisch eines Halogenpropionsäureesters eingesetzt wird.

8. Verfahren nach einem der Ansprüche 1 bis 7, dadurch gekennzeichnet, daß als organisches Lösungsmittel ein Ether eingesetzt wird.

**9.** Verfahren nach einem der Ansprüche 1 bis 8, dadurch gekennzeichnet, daß als Hydrolase eine Lipase eingesetzt wird.

**10.** Verfahren nach einem der Ansprüche 1 bis 9, dadurch gekennzeichnet, daß die Hydrolase an einem Träger adsorbiert eingesetzt wird.

| **EINSCHLÄGIGE DOKUMENTE** | | EP 92106039.8 |

| Kategorie | Kennzeichnung des Dokuments mit Angabe, soweit erforderlich, der maßgeblichen Teile | Betrifft Anspruch | KLASSIFIKATION DER ANMELDUNG (Int. Cl⁵) |
|---|---|---|---|
| X | DD - A - 260 085 (MASSACHUSETTS INSTITUTE OF TECHNOLOGY) * Anspruch 1 * -- | 1,6-9 | C 12 P 41/00 C 12 P 7/40 |
| X | DD - A - 258 625 (MASSACHUSETTS INSTITUTE OF TECHNOLOGY) * Anspruch 1 * -- | 1,6-9 | |
| X | EP - A - 0 257 716 (STAUFFER CHEMICAL COMPANY) * Patentansprüche * ---- | 1,6-9 | |

| | RECHERCHIERTE SACHGEBIETE (Int. Cl⁵) |
|---|---|
| | C 12 P |

Der vorliegende Recherchenbericht wurde für alle Patentansprüche erstellt.

| Recherchenort | Abschlußdatum der Recherche | Prüfer |
|---|---|---|
| WIEN | 08-07-1992 | WOLF |